# EUROPÄISCHE PATENTSCHRIFT

(11) **EP 0 842 155 B1**
(45) Veröffentlichungstag und Bekanntmachung des Hinweises auf die Patenterteilung: **09.04.2003**
(21) Anmeldenummer: 96926347.4
(22) Anmeldetag: 15.07.1996
(51) Int. Cl.: C07D 239/54, C07D 405/12, A01N 43/54

(54) **SUBSTITUIERTE AMINOURACILE**
SUBSTITUTED AMINOURACILS
AMINO-URACILES SUBSTITUES

(30) Priorität: 28.07.1995 DE 19527570
(43) Veröffentlichungstag der Anmeldung: 20.05.1998
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); SCHALLNER, Otto, D-40789 Monheim (DE); DOLLINGER, Markus, D-51381 Leverkusen (DE); SANTEL, Hans-Joachim, D-51371 Leverkusen (DE)
(86) Internationale Anmeldenummer: EP9603088
(87) Internationale Veröffentlichungsnummer: WO97005116

(56) Entgegenhaltungen:
- EP-A- 0 438 209
- EP-A- 0 517 181
- WO-A-96/24590
- DE-A- 19 500 439

## Beschreibung

Die Erfindung betrifft substituierte Aminouracile, Verfahren zu ihrer Herstellung, ihre Verwendung als Herbizide.

Es ist bekannt, daß bestimmte substituierte Uracile herbizide Eigenschaften aufweisen (vgl. EP 408382/US 5084084/US 5127935/US5154755, EP 563384, EP 648749, WO 91/00278, US 4979982, US 5169430). Diese Verbindungen haben jedoch bisher keine nennenswerte Bedeutung erlangt.

Es wurden nun die substituierten Aminouracile der allgemeinen Formel (I) gefunden 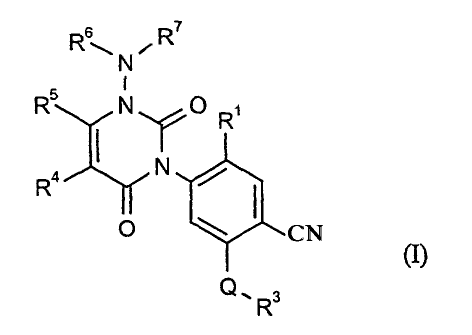 in welcher
- Q: für O, S, SO oder SO₂ steht,
- R¹: für Wasserstoff, Cyano oder Halogen steht,
- R³: für jeweils gegebenenfalls substituiertes Aryl oder Heterocyclyl steht,
- R⁴: für Wasserstoff, Halogen oder für jeweils gegebenenfalls substituiertes Alkyl oder Alkoxy steht,
- R⁵: für gegebenenfalls substituiertes Alkyl steht,
- R⁶: für Wasserstoff oder für jeweils gegebenenfalls substituiertes Alkyl, Alkenyl oder Alkinyl steht, und
- R⁷: für Wasserstoff oder für jeweils gegebenenfalls substituiertes Alkyl, Alkenyl oder Alkinyl steht.

Man erhält die substituierten Aminouracile der allgemeinen Formel (I), wenn man
(a) substituierte Uracile der allgemeinen Formel (II) 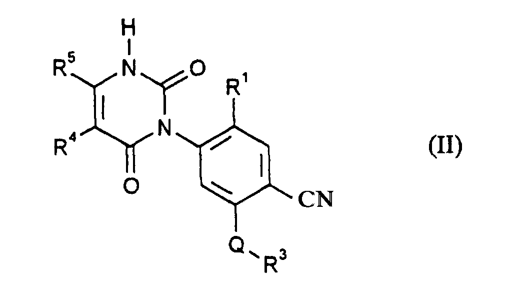 in welcher
   - Q, R¹, R³, R⁴ und R⁵: die oben angegebenen Bedeutungen haben,
   mit einem elektrophilen Aminierungsmittel gegebenenfalls in Gegenwart eines Reaktionshilfsmittels und gegebenenfalls in Gegenwart eines Verdünnungsmittels umsetzt,
   oder wenn man
(b) Aminouracile der allgemeinen Formel (Ib) 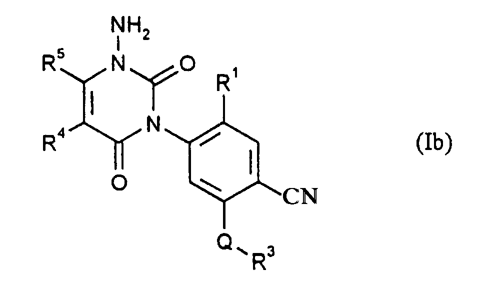 in welcher
   - Q, R¹, R³, R⁴ und R⁵: die oben angegebenen Bedeutungen haben,
   mit einem Alkylierungsmittel der Formel (IIIa) und/oder der Formel (IIIb)

   X-R⁷ (IIIa) X-R⁶ (IIIb)

   in welchen
   - R⁷ und R⁶: die oben angegebenen Bedeutungen haben und
   - X: für Halogen oder die Gruppierung -O-SO₂-O-R⁷ bzw. -O-SO₂-O-R⁶ steht,
   gegebenenfalls in Gegenwart eines Reaktionshilfsmittel und gegebenenfalls ir Gegenwart eines Verdünnungsmittels umsetzt,
   oder wenn man
(c) Aminouracile der allgemeine Formel (IV) 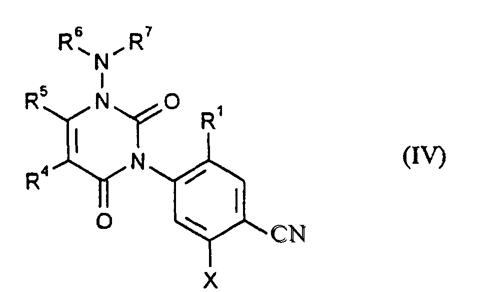 in welcher
   - R¹, R⁴, R⁵, R⁶ und R⁷: die oben angegebenen Bedeutungen haben und
   - X: für Halogen steht,
   mit nucleophilen Verbindungen der allgemeinen Formel (V)

   M-Q-R³ (V)

   in welcher
   - Q und R³: die oben angegebenen Bedeutungen haben und
   - M: für Wasserstoff oder ein Metalläqivalent steht,
   gegebenenfalls in Gegenwart eines Reaktionshilfsmittels und gegebenenfalls in Gegenwart eines Verdünnungsmittels umsetzt

Die substituierten Aminouracile der allgemeinen Formel (I) zeichnen sich durch starke herbizide Eigenschaften aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- Q: für O, S, SO oder SO₂ steht,
- R¹: für Wasserstoff, Cyano, Fluor oder Chlor steht,
- R³: für jeweils gegebenenfalls durch Cyano, Carboxy, Nitro, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl oder C₁-C₄-Alkoxy-carbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl mit 6 oder 10 Kohlenstoffatomen steht,
- R³: weiterhin für jeweils gegebenenfalls durch Cyano, Carboxy, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Furyl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,
- R⁴: für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
- R⁵: für gegebenenfalls durch Fluor und/oder Chlor substituiertes Alkyl mit 1 bis 4 Kohlenstoffatomen steht,
- R⁶: für Wasserstoff oder für jeweils gegebenenfalls durch Fluor, Chlor oder C₁-C₄-Alkoxy substituiertes Alkyl, Alkenyl oder Alkinyl mit jeweils bis zu 6 Kohlenstoffatomen steht, und
- R⁷: für Wasserstoff oder für jeweils gegebenenfalls durch Fluor, Chlor oder C₁-C₄-Alkoxy substituiertes Alkyl, Alkenyl oder Alkinyl mit jeweils bis zu 6 Kohlenstoffatomen steht.

Die Erfindung betrifft insbesondere Verbindungen der Formel (I), in welcher
- Q: für O, S, SO oder SO₂ steht,
- R¹: für Wasserstoff, Fluor oder Chlor steht,
- R³: für jeweils gegebenenfalls durch Cyano, Carboxy, Nitro, Carbamoyl; Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl substituiertes Phenyl, oder für jeweils gegebenenfalls durch Cyano, Carboxy, Carbamoyl, Thiocarbamoyl, Fluor, Chlor, Brom, Methyl, Ethyl, n- oder i-Propyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl, Phenyl, Phenoxy oder Phenylthio substituiertes Furyl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,
- R⁴: für Wasserstoff, Fluor, Chlor, Brom oder Methyl steht,
- R⁵: für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Difluorethyl, Chlorfluorethyl, Chlordifluorethyl, Fluordichlorethyl, Trifluorethyl, Tetrafluorethyl, Chlortrifluorethyl oder Pentafluorethyl steht,
- R⁶: für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl oder Butinyl steht, und
- R⁷: für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl oder Butinyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

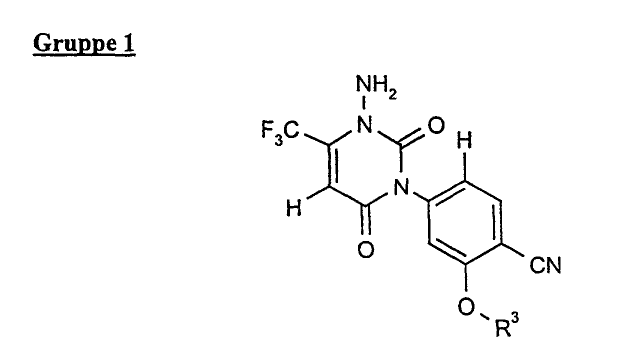

R³ hat hierbei beispielhaft die in der nachstehenden Aufzählung angegebenen Bedeutungen:
Phenyl, Cyanophenyl, Carboxyphenyl, Nitrophenyl, Fluorphenyl, Chlorphenyl, Bromphenyl, Methylphenyl, Trifluormethylphenyl, Methoxyphenyl, Difluormethoxyphenyl, Trifluormethoxyphenyl, Methoxycarbonylphenyl, Ethoxycarbonylphenyl, Furyl, Thienyl, Tetrahydrothienyl, Oxetanyl, Oxazolyl, Isoxazolyl.

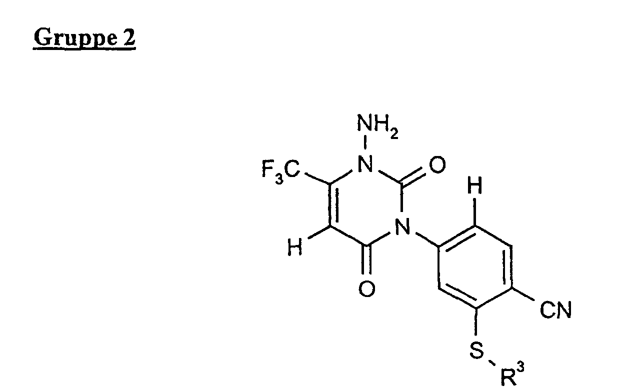

R³ hat hierbei beispielhaft die oben in Gruppe 1 aufgeführten Bedeutungen.

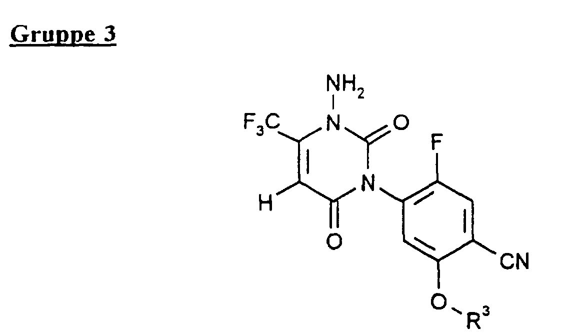

R³ hat hierbei beispielhaft die oben in Gruppe 1 aufgeführten Bedeutungen.

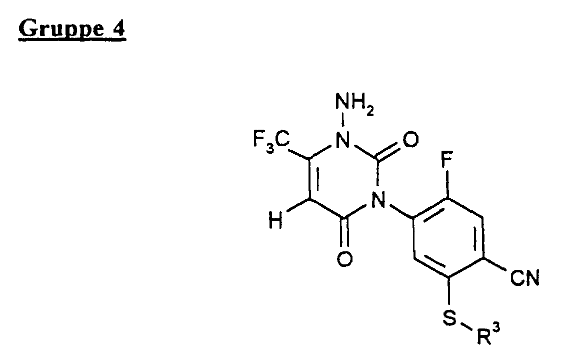

R³ hat hierbei beispielhaft die oben in Gruppe 1 aufgeführten Bedeutungen.

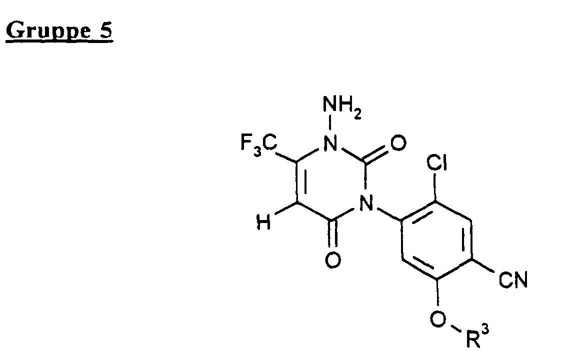

R³ hat hierbei beispielhaft die oben in Gruppe 1 aufgeführten Bedeutungen.

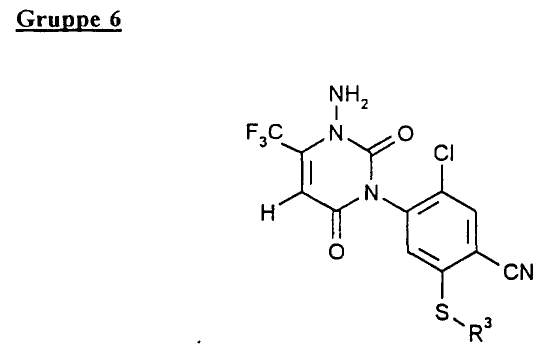

R³ hat hierbei beispielhaft die oben in Gruppe 1 aufgeführten Bedeutungen.

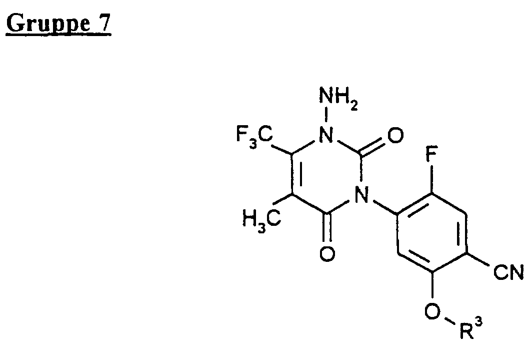

R³ hat hierbei beispielhaft die oben in Gruppe 1 aufgeführten Bedeutungen.

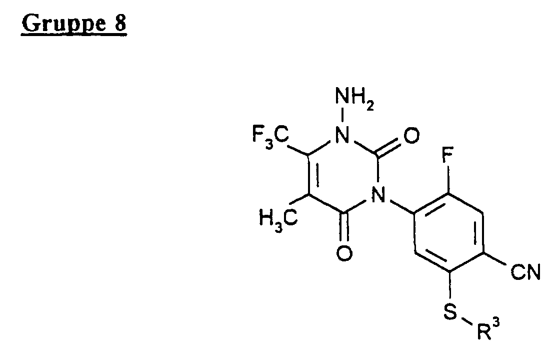

R³ hat hierbei beispielhaft die oben in Gruppe 1 aufgeführten Bedeutungen.

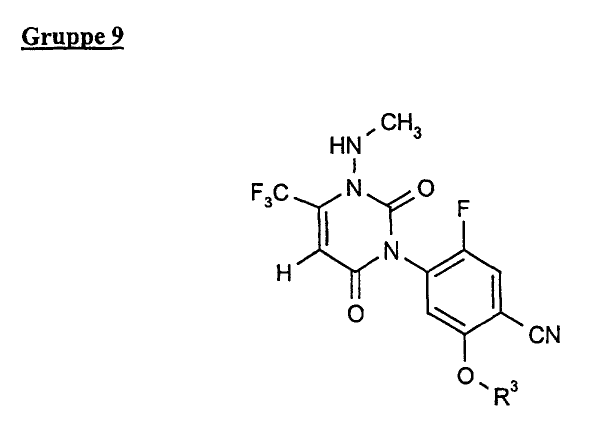

R³ hat hierbei beispielhaft die oben in Gruppe 1 aufgeführten Bedeutungen.

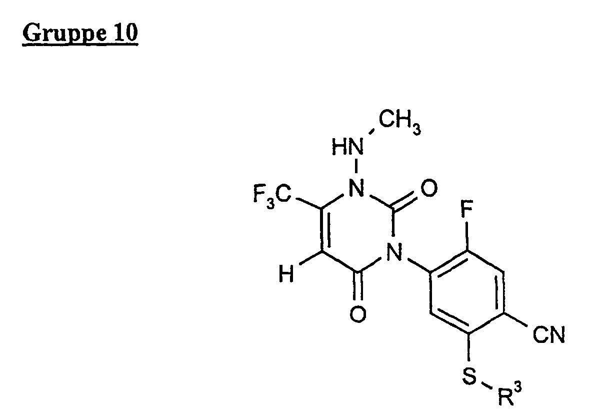

R³ hat hierbei beispielhaft die oben in Gruppe 1 aufgeführten Bedeutungen.

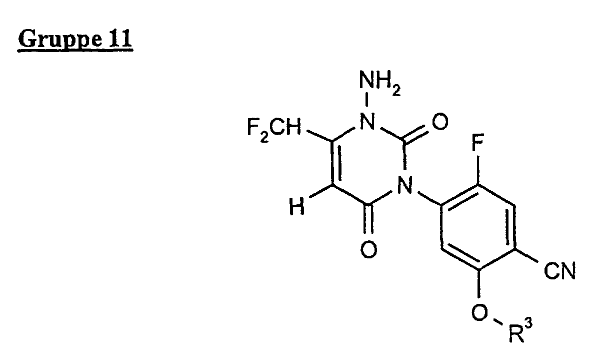

R³ hat hierbei beispielhaft die oben in Gruppe 1 aufgeführten Bedeutungen.

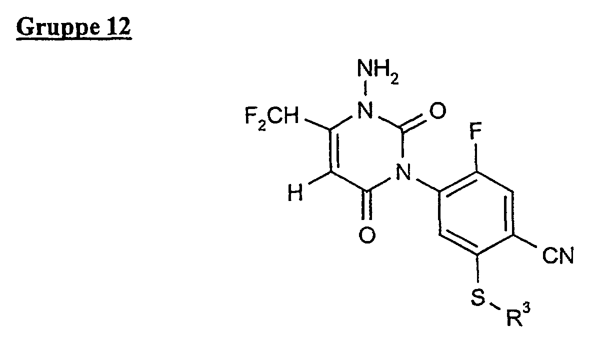

R³ hat hierbei beispielhaft die oben in Gruppe 1 aufgeführten Bedeutungen.

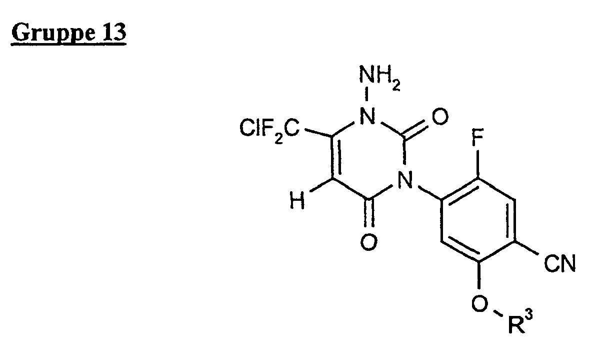

R³ hat hierbei beispielhaft die oben in Gruppe 1 aufgeführten Bedeutungen.

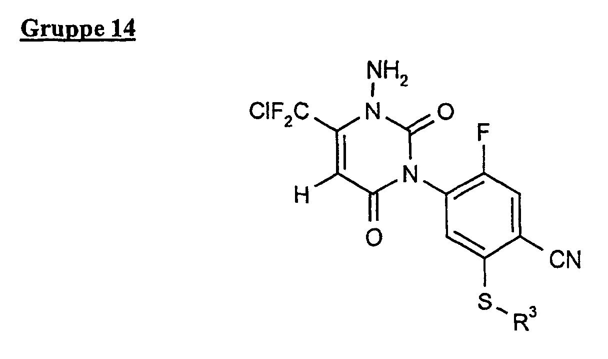

R³ hat hierbei beispielhaft die oben in Gruppe 1 aufgeführten Bedeutungen.

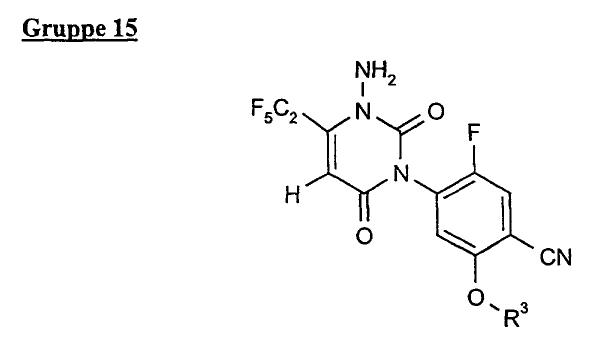

R³ hat hierbei beispielhaft die oben in Gruppe 1 aufgeführten Bedeutungen.

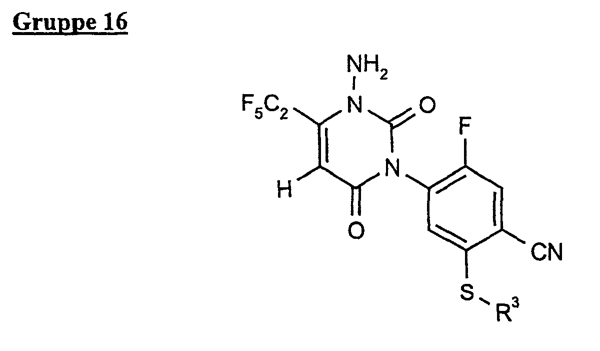

R³ hat hierbei beispielhaft die oben in Gruppe 1 aufgeführten Bedeutungen.

Die beim erfindungsgemäßen Verfahren (a) zur Herstellung der Verbindungen der Formel (I) als Ausgangsstoffe zu verwendenden substituierten Uracile sind durch die Formel (II) allgemein definiert. In der Formel (II) haben Q, R¹, R³, R⁴ und R⁵ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für Q, R¹, R³, R⁴ und R⁵ angegeben wurden.

Die Ausgangsstoffe der Formel (II) sind bekannt und/oder können nach bekannten Verfahren hergestellt werden (vgl. EP 408382, Herstellungsbeispiele).

Das erfindungsgemäße Verfahren (a) wird unter Verwendung eines elektrophilen Aminierungsmittels durchgeführt. Es können hierbei die üblichen elektrophilen Aminierungsmittel eingesetzt werden. Als Beispiele hierfür seien 1-Aminooxy-2,4-dinitro-benzol (2,4-Dinitro-phenyl-hydroxylamin) und Hydroxylamin-O-sulfonsäure genannt.

Die beim erfindungsgemäßen Verfahren (b) zur Herstellung der Verbindungen der Formel (I) als Ausgangsstoffe zu verwendenden Aminouracile sind durch die Formel (Ib) allgemein definiert. In der Formel (Ib) haben Q, R¹, R³, R⁴ und R⁵ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für Q, R¹, R³, R⁴ und R⁵ angegeben wurden.

Die Ausgangsstoffe der Formel (Ib) sind erfindungsgemäße Verbindungen; sie können nach den erfindungsgemäßen Verfahren (a) hergestellt werden.

Die beim erfindungsgemäßen Verfahren (b) zur Herstellung der Verbindungen der Formel (I) weiter als Ausgangsstoffe zu verwendenden Alkylierungsmittel sind durch die Formeln (IIIa) und (IIIb) allgemein definiert. In den Formeln (IIIa) und (IIIb) haben R⁷ und R⁶ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R⁷ und R⁶ angegeben wurde; X steht vorzugsweise für Fluor, Chlor, Brom oder Iod, insbesondere für Chlor, Brom oder Iod.

Die beim erfindungsgemäßen Verfahren (c) zur Herstellung der Verbindungen der Formel (I) als Ausgangsstoffe zu verwendenden Aminouracile sind durch die Formel (IV) allgemein definiert. In der Formel (IV) haben R¹, R⁴, R⁵, R⁶ und R⁷ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R⁴, R⁵, R⁶ und R⁷ angegeben wurden; X steht vorzugsweise fur Fluor, Chlor oder Brom, insbesondere für Fluor oder Chlor.

Die Ausgangsstoffe der Formel (IV) sind bekannt und/oder können nach bekannten Verfahren hergestellt werden (vgl. EP 648749).

Die beim erfindungsgemäßen Verfahren (c) zur Herstellung der Verbindungen der Formel (I) weiter als Ausgangsstoffe zu verwendenden nucleophilen Verbindungen sind durch die Formel (V) allgemein definiert. In der Formel (V) haben Q und R³ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für Q und R³ angegeben wurden; M steht vorzugsweise für Lithium, Natrium, Kalium.

Die Ausgangsstoffe der Formel (V) sind bekannte Synthesechemikalien.

Die erfindungsgemäßen Verfahren (a), (b) und (c) zur Herstellung der Verbindungen der Formel (I) werden vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall-acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8 Diazabicyclo[5,4,0]-undec-7-en (DBU)

Die erfindungsgemäßen Verfahren (a), (b) und (c) zur Herstellung der Verbindungen der Formel (I) werden vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-tbutylether (MTBE), Ethyl-t-butylether, Methyl-t-pentylether (TAME), Ethyl-tpentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -i-propylester, -n-, -i- oder -s-butylester; Sulfoxide, wie beispielsweise Dimethylsulfoxid; Alkanole, wie beispielsweise Methanol, Ethanol, n-oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchführung der erfindungsgemäßen Verfahrens (a), (b) und (c) in einem größeren Bereich variiert werden. Im allgemeinen arbeitet man bei Temperaturen zwischen -20°C und +150°C, vorzugsweise zwischen 0°C und 120°C.

Die erfindungsgemäßen Verfahren (a), (b) und (c) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahrens (a), (b) und (c) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen der Formel (I) eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die erfindungsgemäßen Verbindungen der Formel (I) zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Losungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyanin-farbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuronmethyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemaßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

Entsprechend der allgemeinen Beschreibung der erfindungsgemäßen Herstellungsverfahren können beispielsweise auch die in der nachstehenden Tabelle 1 aufgerührten Verbindungen der Formel (I) hergestellt werden.

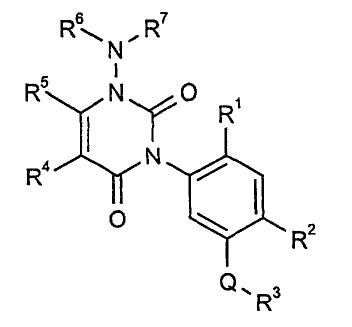

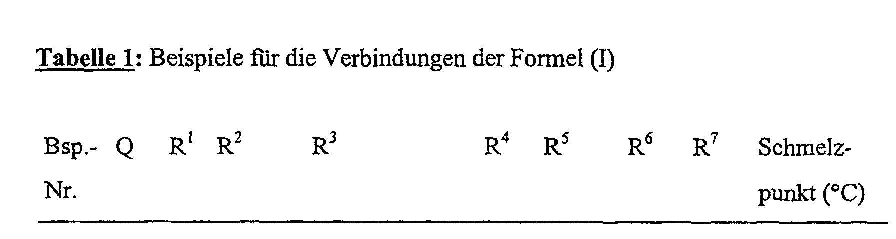

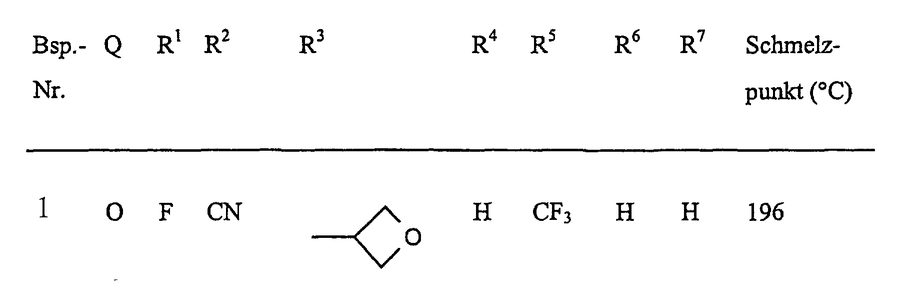

### Ausgangsstoffe der Formel (II):

### Beispiel (II-1)

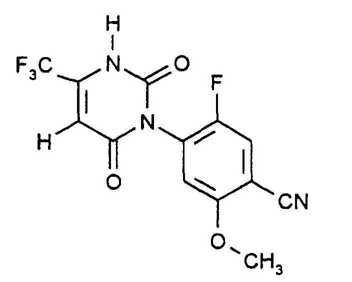

Eine Mischung aus 3,8 g (12 mMol) 1-(4-Cyano-2,5-difluor-phenyl)-3,6-dihydro-2,6-dioxo-4-trifluormethyl-1(2H)-pyrimidin, 1,5 g (21 mMol) Natriummethylat und 50 ml N-Methyl-pyrrolidon wird 36 Stunden bei 130°C gerührt. Nach Abkühlen der Mischung und Verdünnen mit Essigsäureethylester auf etwa das dreifache Volumen wird mit Wasser gewaschen und mittels Chromatographie über eine Kieselgelsäule (Cyclohexan/Essigsäureethylester, Vol.: 1/1) das Produkt isoliert.

Man erhält 1,6 g (41% der Theorie) 1-(4-Cyano-2-fluor-5-methoxy-phenyl)-3,6-dihydro-2,6-dioxo-4-trifluormethyl-1(2H)-pyrimidin vom Schmelzpunkt 114°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten.
- 0 % =: keine Wirkung (wie unbehandelte Kontrolle)
- 100 % =: totale Vernichtung

In diesem Test zeigt beispielsweise die Verbindung gemäß Herstellungsbeispiel 1 bei einer Aufwandmenge von 125 g/ha praktisch vollständige Abtötung einer großen Zahl von Unkräutern, wie z.B. Alopecurus (100 %), Avena (100 %), Cyperus (100 %), Setaria (100 %), Abutilon (100 %), Amaranthus (100 %), Galium (100 %), Sinapis (100 %), Xanthium (100 %).

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigt beispielsweise die Verbindung gemäß Herstellungsbeispiel 1 bei einer Aufwandmenge von 125 g/ha praktisch vollständige Abtötung einer großen Zahl von Unkräutern, wie z.B. Alopecurus (100 %), Avena (100 %), Cyperus (100 %), Setaria (100 %), Abutilon (100 %), Amaranthus (100 %), Galium (100 %), Sinapis (100 %), Xanthium (100 %).

## Patentansprüche

1. Substituierte Aminouracile der allgemeinen Formel (I) 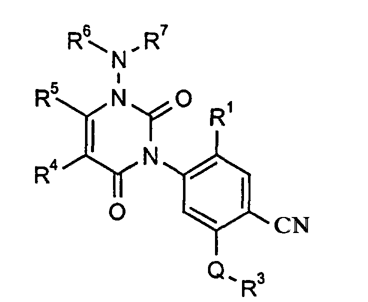 in welcher
Q für O, S, SO oder SO₂ steht,
R¹ für Wasserstoff, Cyano, Fluor oder Chlor steht,
R³ für jeweils gegebenenfalls durch Cyano, Carboxy, Nitro, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl oder C₁-C₄-Alkoxy-carbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl mit 6 oder 10 Kohlenstoffatomen steht, oder
R³ weiterhin für jeweils gegebenenfalls durch Cyano, Carboxy, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl oder C₁-C₄-Alkoxy-carbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Furyl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,
R⁴ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
R⁵ für gegebenenfalls durch Fluor und/oder Chlor substituiertes Alkyl mit 1 bis 4 Kohlenstoffatomen steht,
R⁶ für Wasserstoff oder für jeweils gegebenenfalls durch Fluor, Chlor oder C₁-C₄-Alkoxy substituiertes Alkyl, Alkenyl oder Alkinyl mit jeweils bis zu 6 Kohlenstoffatomen steht, und
R⁷ für Wasserstoff oder für jeweils gegebenenfalls durch Fluor, Chlor oder C₁-C₄-Alkoxy substituiertes Alkyl, Alkenyl oder Alkinyl mit jeweils bis zu 6 Kohlenstoffatomen steht.

2. Substituierte Aminouracile der allgemeinen Formel (I) gemäß Anspruch 1, **dadurch gekennzeichnet, daß**
Q für O, S, SO oder SO₂ steht,
R¹ für Wasserstoff, Fluor oder Chlor steht,
R³ für jeweils gegebenenfalls durch Cyano, Carboxy, Nitro, Carbamoyl, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl substituiertes Phenyl, oder für jeweils gegebenenfalls durch Cyano, Carboxy, Carbamoyl, Thiocarbamoyl, Fluor, Chlor, Brom, Methyl, Ethyl, n- oder i-Propyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl, Phenyl, Phenoxy oder Phenylthio substituiertes Furyl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,
R⁴ für Wasserstoff, Fluor, Chlor, Brom oder Methyl steht,
R⁵ für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Difluorethyl, Chlorfluorethyl, Chlordifluorethyl, Fluordichlorethyl, Trifluorethyl, Tetrafluorethyl, Chlortrifluorethyl oder Pentafluorethyl steht,
R⁶ für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl oder Butinyl steht, und
R⁷ für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl oder Butinyl steht.

3. Verfahren zur Herstellung substituierter Aminouracile der allgemeinen Formel (I) 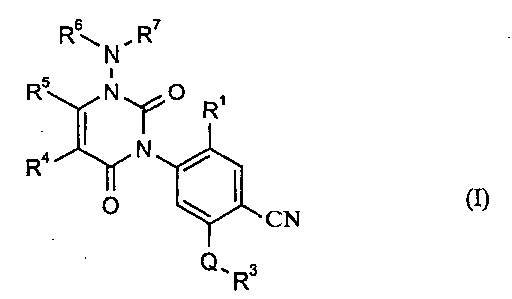 in welcher
Q, R¹, R³, R⁴, R5, R⁶ und R⁷ d ie in Anspruch 1 genannten Bedeutungen haben,
**dadurch gekennzeichnet, daß** man
(a) substituierte Uracile der allgemeinen Formel (II) 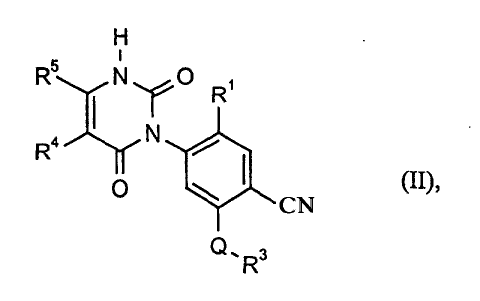 in welcher
Q, R¹, R³, R⁴ und R⁵ die in Anspruch 1 angegebenen Bedeutungen haben,
mit einem elektrophilen Aminierungsmittel gegebenenfalls in Gegenwart eines Reaktionshilfsmittels und gegebenenfalls in Gegenwart eines Verdünnungsmittels umsetzt,
oder daß man
(b) Aminouracile der allgemeinen Formel (Ib) 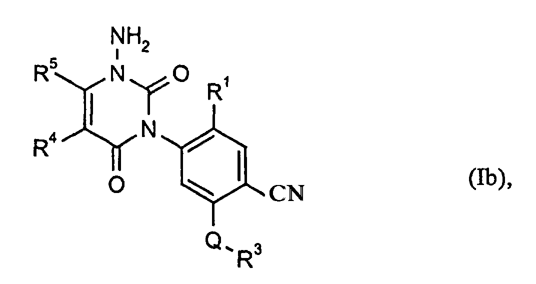 in welcher
Q, R¹, R³, R⁴ und R⁵ die in Anspruch 1 angegebenen Bedeutungen haben,
mit einem Alkylierungsmittel der Formel (IIIa) und/oder der Formel (IIIb)
X-R⁷ (IIIa) X-R⁶ (IIIb)
in welchen
R⁷ und R⁶ die in Anspruch 1 angegebenen Bedeutungen haben und
X für Halogen oder die Gruppierung -O-SO₂-O-R⁷ bzw. -O-SO₂-O-R⁶ steht,
gegebenenfalls in Gegenwart eines Reaktionshilfsmittel und gegebenenfalls in Gegenwart eines Verdünnungsmittels umsetzt,
oder daß man
(c) Aminouracile der allgemeinen Formel (IV) 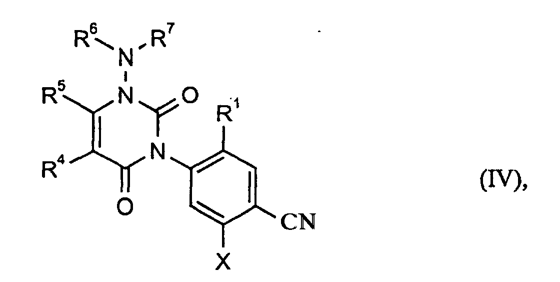 in welcher
R¹, R⁴, R⁵, R⁶ und R⁷ die in Anspruch 1 angegebenen Bedeutungen haben und
X für Halogen steht,
mit nucleophilen Verbindungen der allgemeinen Formel (V)
M-Q-R³ (V)
in welcher
Q und R³ die in Anspruch 1 angegebenen Bedeutungen haben und
M für Wasserstoff oder ein Metalläqivalent steht,
gegebenenfalls in Gegenwart eines Reaktionshilfsmittels und gegebenenfalls in Gegenwart eines Verdünnungsmittels umsetzt.

4. Herbizide Mittel, **gekennzeichnet durch** einen Gehalt an mindestens einem substituierten Aminouracil der allgemeinen Formel (I) gemäß den Ansprüchen 1 und 2.

5. Verfahren zur Bekämpfung von unerwünschten Pflanzen, **dadurch gekennzeichnet, daß** man substituierte Aminouracile der allgemeinen Formel (I) gemäß den Ansprüchen 1 und 2 auf unerwünschte Pflanzen und/oder ihren Lebensraum einwirken läßt.

6. Verwendung von substituierten Aminouracilen der allgemeinen Formel (I) gemäß der Ansprüche 1 und 2 zur Bekämpfung von unerwünschten Pflanzen.

7. Verfahren zur Herstellung von herbiziden Mitteln, **dadurch gekennzeichnet, daß** man substituierte Aminouracile der allgemeinen Formel (I) gemäß der Ansprüche 1 und 2 mit Streckmitteln und/oder oberflächenaktiven Substanzen vermischt.

## Claims

1. Substituted aminouracils of the general formula (I) 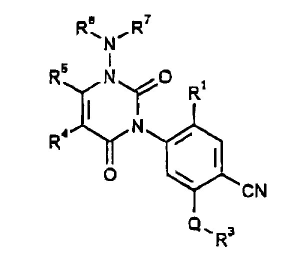 in which
Q represents O, S, SO or SO₂,
R¹ represents hydrogen, cyano, fluorine or chlorine,
R³ represents aryl of 6 or 10 carbon atoms, each of which is optionally substituted by cyano, carboxy, nitro, carbamoyl, thiocarbamoyl, fluorine, chlorine, bromine, by C₁-C₄-alkyl, C₁-C₄-alkoxy, C₁-C₄-alkylthio, C₁-C₄-alkylsulphinyl, C₁-C₄-alkylsulphonyl, C₁-C₄-alkyl-carbonyl or C₁-C₄-alkoxy-carbonyl (each of which is optionally substituted by fluorine and/or chlorine), by phenyl, phenoxy or phenylthio (each of which is optionally substituted by fluorine, chlorine, bromine, cyano, methyl, methoxy, trifluoromethyl and/or trifluoromethoxy), or
R³ furthermore represents furyl, thienyl, tetrahydrothienyl, oxetanyl, thietanyl, oxazolyl, isoxazolyl, thiazolyl, oxadiazolyl, thiadiazolyl, pyrazolyl, pyridinyl, pyrimidinyl, triazinyl, indolyl, quinolinyl or quinoxalinyl, each of which is optionally substituted by cyano, carboxy, carbamoyl, thiocarbamoyl, fluorine, chlorine, bromine, by C₁-C₄-alkyl, C₁-C₄-alkoxy, C₁-C₄-alkylthio, C₁-C₄-alkylsulphinyl, C₁-C₄-alkylsulphonyl, C₁-C₄-alkyl-carbonyl or C₁-C₄-alkoxy-carbonyl (each of which is optionally substituted by fluorine and/or chlorine), by phenyl, phenoxy or phenylthio (each of which is optionally substituted by fluorine, chlorine, bromine, cyano, methyl, methoxy, trifluoromethyl and/or trifluoromethoxy),
R⁴ represents hydrogen, fluorine, chlorine, bromine or represents in each case optionally fluorine- and/or Chlorine-substituted alkyl or alkoxy having in each case 1 to 4 carbon atoms,
R⁵ represents optionally fluorine- and/or chlorine-substituted alkyl having 1 to 4 carbon atoms,
R⁶ represents hydrogen or represents in each case optionally fluorine-, chlorine- or C₁-C₄-alkoxy-substituted alkyl, alkenyl or alkinyl having in each case up to 6 carbon atoms, and
R⁷ represents hydrogen or represents in each case optionally fluorine-, chlorine- or C₁-C₄-alkoxy-substituted alkyl, alkenyl or alkinyl having in each case up to 6 carbon atoms.

2. Substituted aminouracils of the general formula (I) according to Claim 1, **characterized in that**
Q represents 0, S, SO or SO₂,
R¹ represents hydrogen, fluorine or chlorine,
R³ represents in each case optionally cyano-, carboxy-, nitro-, carbamoyl-, thiocarbamoyl-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, methylthio-, ethylthio-, methylsulphinyl-, ethylsulphinyl-, methylsulphonyl-, ethylsulphonyl-, acetyl-, propionyl-, methoxycarbonyl-, ethoxycarbonyl-, n- or i-propoxycarbonyl-substituted phenyl, or represents in each case optionally cyano-, carboxy-, carbamoyl-, thiocarbamoyl-, fluorine-, chlorine-, bromine-, methyl-, ethyl-, n- or i-propyl-, methoxy-, ethoxy-, n- or i-propoxy-, methylthio-, ethylthio-, methylsulphinyl-, ethylsulphinyl-, methylsulphonyl-, ethylsulphonyl-, acetyl-, propionyl-, methoxycarbonyl-, ethoxycarbonyl-, n- or i-propoxycarbonyl-, phenyl-, phenoxy- or phenylthio-substituted substituted furyl, thienyl, tetrahydrothienyl, oxetanyl, thietanyl, oxazolyl, isoxazolyl, thiazolyl, oxadiazolyl, thiadiazolyl, pyrazolyl, pyridinyl, pyrimidinyl, triazinyl, indolyl, quinolinyl or quinoxalinyl,
R⁴ represents hydrogen, fluorine, chlorine, bromine or methyl,
R⁵ represents methyl, ethyl, difluoromethyl, dichloromethyl, trifluoromethyl, trichloromethyl, chlorodifluoromethyl, fluorodichloromethyl, chloroethyl, fluoroethyl, dichloroethyl, difluoroethyl, chlorofluoroethyl, chlorodifluoroethyl, fluorodichloroethyl, trifluoroethyl, tetrafluoroethyl, chlorotrifluoroethyl or pentafluoroethyl,
R⁶ represents hydrogen or represents in each case optionally fluorine-, chlorine-, methoxy- or ethoxy-substituted methyl, ethyl, n- or i-propyl, n-, i-, s- or t-butyl-, propenyl, butenyl, propinyl or butinyl, and
R⁷ represents hydrogen or represents in each case optionally fluorine-, chlorine-, methoxy- or ethoxy-substituted methyl, ethyl, n- or i-propyl, n-, i-, s- or t-butyl, propenyl, butenyl, propinyl or butinyl.

3. Process for preparing substituted aminouracils of the general formula (I) 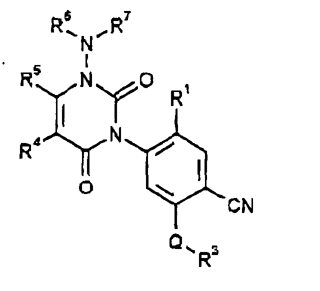 in which
Q, R¹, R³, R⁴, R⁵, R⁶ and R⁷ are each as defined in Claim 1,
**characterized in that**
a) substituted uracils of the general formula (II) 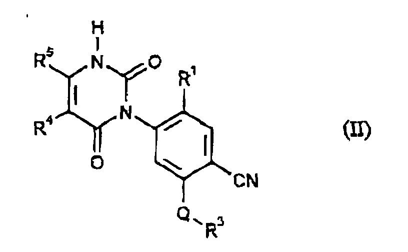 in which
Q, R¹, R³, R⁴, and R⁵ are each as defined in Claim 1,
are reacted with an electrophilic aminating agent, if appropriate in the presence of a reaction auxiliary, and if appropriate in the presence of a diluent,
or
(b) aminouracils of the general formula (Ib) 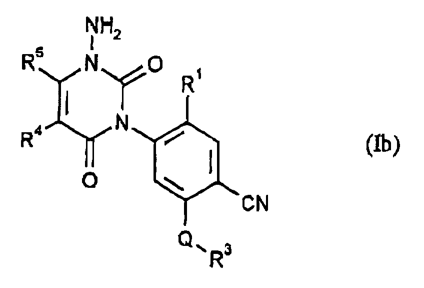 in which
Q, R¹, R³, R⁴ and R⁵ are each as defined in Claim 1,
are reacted with an alkylating agent of the formula (IIIa) and/or of the formula (IIIb)
X-R⁷ (IIIa) X-R⁶ (IIIb)
in which
R⁷ and R⁶ are each as defined in Claim 1 and
X represents hydrogen or a metal equivalent,
if appropriate in the presence of a reaction auxiliary and if appropriate in the presence of a diluent.

4. Herbicides, **characterized in that** they comprise at least one substituted aminouracil of the general formula (I) according to Claims 1 and 2.

5. Method for controlling undesirable plants, **characterized in that** substituted aminouracils of the general formula (I) according to Claims 1 and 2 are allowed to act on undesirable plants and/or their habitat.

6. Use of substituted aminouracils of the general formula (I) according to Claims 1 and 2 for controlling undesirable plants.

7. Process for preparing herbicides, **characterized in that** substituted aminouracils of the general formula (I) according to Claims 1 and 2 are mixed with extenders and/or surfactants.

## Revendications

1. Amino-uraciles substitués de formule générale (I) 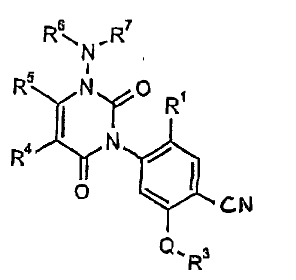 dans laquelle
Q représente O, S, SO ou SO₂,
R¹ est l'hydrogène, un reste cyano, le fluor ou le chlore,
R³ est un reste aryle ayant 6 ou 10 atomes de carbone, éventuellement substitué dans chaque cas par un radical cyano, carboxy, nitro, carbamoyle, thiocarbamoyle, fluoro, chloro, bromo, par un radical alkyle en C₁ à C₄, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle ou (alkoxy en C₁ à C₄)-carbonyle (dont chacun est éventuellement substitué par du fluor et/ou du chlore), par un radical phényle, phénoxy ou phénylthio (dont chacun est éventuellement substitué par du fluor, du chlore, du brome, un radical cyano, méthyle, méthoxy, trifluorométhyle et/ou trifluorométhoxy), ou bien
R³ est un reste furyle, thiényle, tétrahydrothiényle, oxétannyle, thiétannyle, oxazolyle, isoxazolyle, thiazolyle, oxadiazolyle, thiadiazolyle, pyrazolyle, pyridinyle, pyrimidinyle, triazinyle, indolyle, quinolinyle ou quinoxalinyle éventuellement substitué dans chaque cas par un radical cyano, carboxy, carbamoyle, thiocarbamoyle, fluoro, chloro, bromo, par un radical alkyle en C₁ à C₄, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle ou (alkoxy en C₁ à C₄)-carbonyle (dont chacun est éventuellement substitué par du fluor et/ou du chlore), par un radical phényle, phénoxy ou phénylthio (dont chacun est éventuellement substitué par un radical fluoro, chloro, bromo, cyano, méthyle, méthoxy, trifluorométhyle et/ou trifluorométhoxy),
R⁴ est l'hydrogène, le fluor, le chlore ou le brome ou un reste alkyle ou alkoxy ayant chacun 1 à 4 atomes de carbone et dont chacun est éventuellement substitué par du fluor et/ou du chlore,
R⁵ est un reste alkyle ayant 1 à 4 atomes de carbone éventuellement substitué par du fluor et/ou du chlore,
R⁶ est l'hydrogène ou un reste alkyle, alcényle ou alcynyle ayant chacun jusqu'à 6 atomes de carbone et dont chacun est éventuellement substitué par du fluor, du chlore ou un radical alkoxy en C₁ à C₄, et
R⁷ est l'hydrogène ou un reste alkyle, alcényle ou alcynyle ayant chacun jusqu'à 6 atomes de carbone et dont chacun est éventuellement substitué par du fluor, du chlore ou un radical alkoxy en C₁ à C₄.

2. Amino-uraciles substitués de formule générale (I) suivant la revendication 1, **caractérisé en ce que** :
Q représente O, S, SO ou SO₂,
R¹ est l'hydrogène, le fluor ou le chlore,
R³ est un reste phényle éventuellement substitué dans chaque cas par un radical cyano, carboxy, nitro, carbamoyle, thiocarbamoyle, fluoro, chloro, brome, méthyle, éthyle, n-propyle, isopropyle, n-butyle, isobutyle, sec.-butyle, tertio-butyle, trifluorométhyle, méthoxy, éthoxy, n-propoxy, isopropoxy, méthylthio, éthylthio, méthylsulfinyle, éthylsulfinyle, méthylsulfonyle, éthylsulfonyle, acétyle, propionyle, méthoxycarbonyle, éthoxycarbonyle, n-propoxycarbonyle ou isopropoxycarbonyle, ou un reste furyle, thiényle, tétrahydrothiényle, oxétannyle, thiétannyle, oxazolyle, isoxazolyle, thiazolyle, oxadiazolyle, thiadiazolyle, pyrazolyle, pyridinyle, pyrimidinyle, triazinyle, indolyle, quinolinyle ou quinoxalinyle dont chacun est éventuellement substitué par un radical cyano, carboxy, carbamoyle, thiocarbamoyle, fluoro, chloro, bromo, méthyle, éthyle, n-propyle, isopropyle, méthoxy, éthoxy, n-propoxy, isopropoxy, méthylthio, éthylthio, méthylsulfinyle, éthylsulfinyle, méthylsulfonyle, éthylsulfonyle, acétyle, propionyle, méthoxycarbonyle, éthoxycarbonyle, n-propoxycarbonyle, isopropoxycarbonyle, phényle, phénoxy ou phénylthio,
R⁴ est l'hydrogène, le fluor, le chlore, le brome ou un reste méthyle,
R⁵ est un rste méthyle, éthyle, difluoromethyle, dichlorométhyle, trifluorométhyle, trichlorométhyle, chloridifluorométhyle, fluorodichlorométhyle, chloréthyle, fluoréthyle, dichloréthyle, difluoréthyle, chlorofluoréthyle, chlorodifluoréthyle, fluorodichloréthyle, trifluoréthyle, tétrafluoréthyle, chlorotrifluoréthyle ou pentafluoréthyle,
R⁶ est l'hydrogène ou un reste méthyle, éthyle, n-propyle, isopropyle, n-butyle, isobutyle, sec.-butyle, tertio-butyle, propényle, butényle, propynyle ou butynyle dont chacun est éventuellement substitué par un radical fluoro, chloro, méthoxy ou éthoxy, et
R⁷ est l'hydrogène ou un reste méthyle, éthyle, n-propyle, isopropyle, n-butyle, isobutyle, sec.-butyle, tertio-butyle, propényle, butényle, propynyle ou butynyle dont chacun est éventuellement substitué par un radical fluoro, chloro, méthoxy ou éthoxy.

3. Procédé de production d'amino-uraciles substitués de formule générale (I) 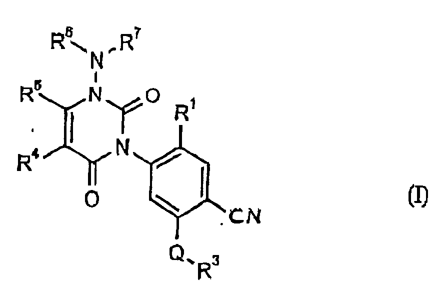 dans laquelle
Q, R¹, R³, R⁴, R⁵, R⁶ et R⁷ ont les définitions indiquées dans la revendication 1,
**caractérisé en ce que** ;
(a) on fait réagir des uraciles substitués de formule générale (II) 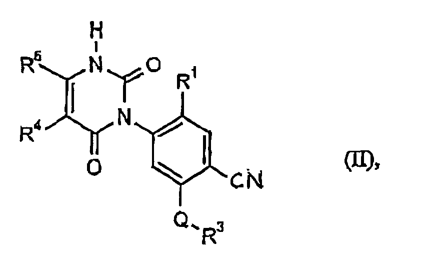 dans laquelle
Q, R¹, R³, R⁴ et R⁵ ont les définitions. indiquées dans la revendication 1,
avec un agent électrophile d'amination, le cas échéant en présence d'un auxiliaire de réaction et en présence éventuelle d'un diluant,
ou **en ce que** :
(b) on fait réagir des amine-uraciles de formule générale (Ib) 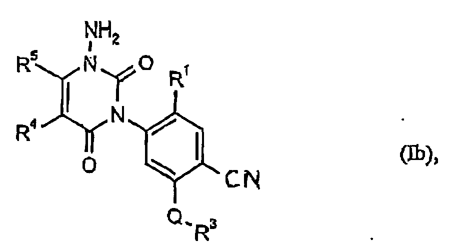 dans laquelle
Q, R¹, R³, R⁴ et R³ ont les définitions indiquées dans la revendication 1,
avec un agent d'alkylation de formule (IIIa) et/ou de formule (IIIb)
X-R⁷ (IIIa) X-R⁶ (IIIb)
formules dans lesquelles
R⁷ et R⁶ ont les définitions indiquées dans la revendication 1 et
X est un halogène ou le groupement -O-SO₂-O-R⁷ ou -O-SO₂-O-R⁶,
le cas échéant en présence d'un auxiliaire de réaction et en présence éventuelle d'un diluant,
ou **en ce que**
(c) on fait réagir des amino-uraciles de formule générale (IV) 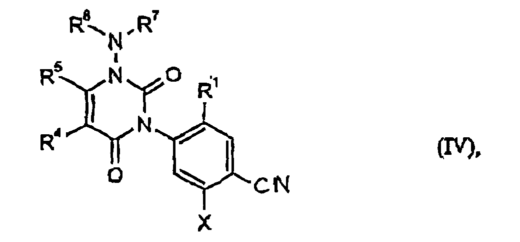 dans laquelle
R¹, R⁴, R⁵, R⁶ et R⁷ ont les définitions indiquées dans la revendication 1 et
X est un halogène,
avec des composés nucléophiles de formule générale (V)
M-Q-R³ (V)
dans laquelle
Q et R³ ont les définitions indiquées dans la revendication 1 et
M est l'hydrogène ou un équivalent de métal,
le cas échéant en présence d'un auxiliaire de réaction et en présence éventuelle d'un diluant.

4. Compositions herbicides, **caractérisées par** une teneur en au moins un amino-uracile substitué de formule générale (I) suivant les revendications 1 et 2.

5. Procédé de lutte contre des plantés indésirables, **caractérisé en ce qu'**on fait réagir des amino-uraciles substitués de formule générale (I) suivant les revendications 1 et 2 sur des plantes indésirables et/ou sur leur milieu.

6. Utilisation d'amine-uraciles substitués de formule générale (I) suivant les revendications 1 et 2 pour la lutte contre des plantes indésirables.

7. Procédé de préparation de compositions herbicides, **caractérisé en ce qu'**on mélange des amine-uraciles substitués de formule générale (I) suivant les revendications 1 et 2 avec des diluants et/ou des agents tensio-actifs.
